**Europäisches Patentamt**

**European Patent Office**

**Office européen des brevets**

(11) Publication number : **0 546 761 A1**

## (12) EUROPEAN PATENT APPLICATION

(21) Application number : **92310998.7**

(51) Int. Cl.⁵ : **C12Q 1/68**

(22) Date of filing : **02.12.92**

(30) Priority : **11.12.91 US 806933**

(43) Date of publication of application :
**16.06.93 Bulletin 93/24**

(84) Designated Contracting States :
**DE FR GB SE**

(71) Applicant : **BECTON, DICKINSON & COMPANY**
**One Becton Drive**
**Franklin Lakes New Jersey 07417-1880 (US)**

(72) Inventor : **Malinowski, Douglas Peter**
**Route 6, Box 696, Dinmocks Mill Road**
**Hillsborough, NC 27278 (US)**
Inventor : **Fraiser, Melinda Susan**
**104 East Maynard Avenue**
**Durham, NS 27704 (US)**
Inventor : **Jurgensen, Steward R.**
**7504 Valley Run Drive**
**Raley, NC 27615 (US)**

(74) Representative : **Ruffles, Graham Keith et al**
**MARKS & CLERK 57-60 Lincoln's Inn Fields**
**London WC2A 3LS (GB)**

(54) **Probes to chlamydia trachomatis.**

(57)    The invention provides methods and nucleic acid probes for detecting and isolating sequences of Chlamydia Trachomatis. The probes can recognize all fifteen serotypes of C. trachomatis.

EP 0 546 761 A1

FIELD OF THE INVENTION

The invention is in the field of molecular biology. In particular, the invention relates to diagnostics involving nucleic acid probes. More particularly, the invention relates to nucleic acid probes for detecting and isolating Chlamydia trachomatis.

BACKGROUND OF THE INVENTION

The genus of bacteria known as Chlamydia contains two species, one of which is Chlamydia trachomatis . C. trachomatis is a bacteria with DNA, RNA, and a cell wall. These bacteria lack the enzymes necessary for oxidative phosphorylation, so they are obligate intracellular parasites for at least a portion of their life cycle. During the extracellular portion of their life cycle, infectious particles, referred to as "elementary bodies," are extremely small. These elementary bodies attach to columnar epithelium of cells and gain entry through phagocytosis. During the intracellular portion of their life cycle, Chlamydiae utilize the adenosine triphosphate (ATP) and amino acids of the cell to replicate via binary fission. New infectious particles are produced that are released with cell lysis.

Genitourinary infections caused by the bacterial pathogen Chlamydia trachomatis are now recognized to be the most common sexually transmitted disease in the United States. It is estimated that three to four million or more cases occur annually, making chlamydial infections more common in the United States than the estimated new cases of gonorrhea, syphilis, and herpes simplex infection combined. The incidents of cervical colonization in the general population is three to five per cent, however, among certain high risk patient populations, cervical carriage rates may approach fifteen to twenty percent.

Chlamydia trachomatis is the leading cause of pneumonia in infants less than six months of age and has replaced gonococcal infections as the leading cause of neonatal conjunctivitis. Chlamydial infection has been found to cause five to ten percent of cases of accute salpingitis in the United States. The majority of cases of accute epididymitis in men under the age of thirty-five are caused by Chlamydia. Infertility has been described as a long-term complication, as a result of scarring and occlusion of the vas deferens. Trachoma is a chronic infection of the conjunctiva (sometimes of the cornea) due to C. trachomatis. Trachoma is the major cause of blindness in humans. Inclusion conjunctivitis is an accute ocular infection due to C. trachomatis that may occur either in neonates or in sexually active adults. Mucopurulent cervicitis, accute urethral syndrome, proctitis, neonatal chlamydial pneumonia, chlamydial pneumonia in immunosuppressed patients, and lymphogranuloma venereum (a venereal disease) are all caused in part by C. trachomatis. In addition, several retrospective studies demonstrate that a significantly higher instance of stillbirth, premature delivery, and neonatal death among pregnant women has been contributed to chlamydial infection.

Fifteen serotypes of Chlamydia trachomatis have been identified. Serotypes A, B, Ba, and C are associated with blinding trachoma in adults and serotypes L1, L2, and L3 are the causitive agents in lymphogranuloma venereum. Serotypes D through K are most commonly found in genitourinary infections.

Today, the definitive laboratory diagnosis for Chlamydia trachomatis infected individuals is isolation using tissue culture from the presumptively infected individuals. The tissue culture methodologies that assure reliable recovery, however, are expensive, cumbersome, stringent, and not suited for large scale. In addition, Papanicolaou's smears are not acceptable for either screening or diagnosis, since their sensitivity ranges from forty to sixty percent among culture positive individuals. Likewise, serologic testing is of little clinical value, since neither seropositives nor seronegatives reliably correlate with infection or absence of infection. Therefore, despite being the scientifically desirable alternative, the cost and labor of tissue culture isolation alone make it an impractical procedure.

Thus it would be desirable to have a detection means for Chlamydia trachomatis that is definitive, cost effective, rapid, and capable of recognizing all fifteen serotypes of C. trachomatis.

SUMMARY OF THE INVENTION

The present invention provides nucleic acid probes and methods that are definitive, rapid, and cost effective for detecting all fifteen serotypes of Chlamydia trachomatis. The invention also provides methods for isolating sequences of all fifteen serotypes of C. trachomatis. In addition, probes of the invention are useful for amplification of sequences to which they hybridize.

Probes of the invention can also be used to construct amino acid sequences for which they encode and antibodies raised to recognize the sequence.

Probes of the invention consist essentially of the nucleic acid sequences in Sequence ID No: 1-21. The probes can locate any or all fifteen serotypes of C. trachomatis. Since the probes of the invention are unique

2

for all fifteen serotypes of <u>C. trachomatis</u>, there is no cross-hybridization to nucleic acid from other gram negative microorganisms.

<u>DETAILED DESCRIPTION</u>

The present invention provides a probe having a sequence consisting essentially of Seq ID No:1, Seq ID No: 2, Seq ID No: 3, Seq ID No: 4, Seq ID No: 5, Seq ID No: 6, Seq ID No: 7, Seq ID No: 8, Seq ID No: 9, Seq ID No: 10, Seq ID No: 11, Seq ID No: 12, Seq ID No: 13, Seq ID No: 14, Seq ID No: 15, Seq ID No: 16, Seq ID No: 17, Seq ID No: 18, Seq ID No: 19, Seq ID No: 20, or Seq ID No: 21, and the modified backbone (includes modified sugar and phosphates groups (e.g. thiophosphates)), modified nucleotide, labelled forms, and ribonucleic acid forms thereof.

A method of amplifying a major outer membrane protein gene which comprises the use of one or more nucleic acid sequences selected from the sequences consisting essentially of Seq ID No:1, Seq ID No: 2, Seq ID No: 3, Seq ID No: 4, Seq ID No: 5, Seq ID No: 6, Seq ID No: 7, Seq ID No: 8, Seq ID No: 9, Seq ID No: 10, Seq ID No: 11, Seq ID No: 12, Seq ID No: 13, Seq ID No: 14, Seq ID No: 15, Seq ID No: 16, Seq ID No: 17, Seq ID No: 18, Seq ID No: 19, Seq ID No: 20, and Seq ID No: 21, and the modified backbone, modified nucleotide, labelled forms, and ribonucleic acid forms thereof is also provided.

Also provided is a method of amplifying nucleic acid and detecting the amplified product which comprises the use of one or more sequences selected from the sequences consisting essentially of Seq ID No:1, Seq ID No: 2, Seq ID No: 3, Seq ID No: 4, Seq ID No: 5, Seq ID No: 6, Seq ID No: 7, Seq ID No: 8, Seq ID No: 9, Seq ID No: 10, Seq ID No: 11, Seq ID No: 12, Seq ID No: 13, Seq ID No: 14, Seq ID No: 15, Seq ID No: 16, Seq ID No: 17, Seq ID No: 18, Seq ID No: 19, Seq ID No: 20, and Seq ID No: 21, and the modified backbone, modified nucleotide, labelled forms, and ribonucleic acid forms thereof.

A kit comprising a nucleic acid sequence selected from the sequences consisting essentially of Seq ID No:1, Seq ID No: 2, Seq ID No: 3, Seq ID No: 4, Seq ID No: 5, Seq ID No: 6, Seq ID No: 7, Seq ID No: 8, Seq ID No: 9, Seq ID No: 10, Seq ID No: 11, Seq ID No: 12, Seq ID No: 13, Seq ID No: 14, Seq ID No: 15, Seq ID No: 16, Seq ID No: 17, Seq ID No: 18, Seq ID No: 19, Seq ID No: 20, and Seq ID No: 21, and the modified backbone, modified nucleotide, labelled forms, and ribonucleic acid forms thereof is also provided.

Also provided is an antibody that recognizes the amino acid sequence encoded by the sequence selected from the sequence consisting essentially of Seq ID No:1, Seq ID No: 2, Seq ID No: 3, Seq ID No: 4, Seq ID No: 5, Seq ID No: 6, Seq ID No: 7, Seq ID No: 8, Seq ID No: 9, Seq ID No: 10, Seq ID No: 11, Seq ID No: 12, Seq ID No: 13, Seq ID No: 14, Seq ID No: 15, Seq ID No: 16, Seq ID No: 17, Seq ID No: 18, Seq ID No: 19, Seq ID No: 20, and Seq ID No: 21.

Probes of the invention are based upon unique nucleic acid sequences in the gene encoding the major outer membrane protein (MOMP) for <u>C. trachomatis</u>. The MOMP gene is present in all fifteen serotypes of <u>C. trachomitis</u>. Therefore, the advantages of a probe based on an MOMP gene include the assurances that the gene encoding the MOMP will be present in all strains and isolates of the microorganism. Likewise, since the gene sequence for <u>C. trachomatis</u> MOMP is unique, there will be no cross-hybridization to nucleic acid from other bacteria. Probes of the invention can detect all 15 serovars of Chlamydia (A,B,Ba,C,D,E,F,G,H,I,J,K,L1,L2, and L3).

The use of probes designed to identify chlamydia based on identification of the MOMP gene overcomes several disadvantages associated with identification based on Chlamydia plasmid or ribosomal RNA. The chlamydia plasmid is cryptic and does not code for any known function. Not all strains are known to contain the plasmid, therefore, diagnosis based on such could easily give false negative results. Identification based on ribosomal RNA could easily lead to false positive results due to cross reactivity with other gram-negative bacteria that have homologous 16S ribosomal RNA genes. Also, RNA copies fluctuate according to antibiotic treatment.

The MOMP gene, however, is essential for the structural integrity of chlamydia and therefore will be present in all strains and isolates of the organism. Due to the unique gene sequence for Chlamydia MOMP, cross hybridization on to genomic DNA from other gram-negative bacteria will not occur.

Sequenced genes of chlamydia MOMP show regions of conserved nucleotide sequences interspersed with regions of hypervariable sequences. Based upon secondary structure formation, some probe sequences will generate less signal in the assay due to interference with hybridization to the target DNA; leading to both inefficient DNA amplifications and inefficient hybridization onto target DNA. However, probes of the invention work well in both amplification and detection.

Probes of the invention hybridize to the following regions of the MOMP gene:
Sequence ID No: 1 corresponds to nucleotides 169-190, Sequence ID No: 2 corresponds to nucleotides 1337-1313, Sequence ID No: 3 corresponds to nucleotides 301-321, Sequence ID No: 4 corresponds to nucleotides

3

321-301, Sequence ID No: 5 corresponds to nucleotides 1216-1241, Sequence ID No: 6 corresponds to nucleotides 1241-1216, Sequence ID No: 7 corresponds to nucleotides 1076-1096, Sequence ID No: 8 corresponds to nucleotides 767-788, Sequence ID No: 9 corresponds to nucleotides 544-566, Sequence ID No: 10 corresponds to nucleotides 718-746, Sequence ID No: 11 corresponds to nucleotides 747-766, Sequence ID No: 12 corresponds to nucleotides 769-786, Sequence ID No: 13 corresponds to nucleotides 767-792, Sequence ID No: 14 corresponds to nucleotides 747-763, Sequence ID No: 15 corresponds to nucleotides 744-766, Sequence ID No: 16 corresponds to nucleotides 795-816, Sequence ID No: 17 corresponds to nucleotides 544-562, Sequence ID No: 18 corresponds to nucleotides 544-571, Sequence ID No: 19 corresponds to nucleotides 718-742, Sequence ID No: 20 corresponds to nucleotides 718-751, and Sequence ID No: 21 corresponds to nucleotides 1199-1268 (with 5'10 base spacer).

Table 1 sets forth probes of the invention (left to right is 5' → 3').

TABLE 1

| | Nucleotide Sequence | Length |
|---|---|---|
| (1) | ATGAAAAAAC TCTTGAAATC GG | 22 |
| (2) | GCATTTACGT GAGCTGCTCT CTCAT | 25 |
| (3) | GGAGATCCTT GCGATCCTTG CC | 22 |
| (4) | GCAAAGATCG CAAGGATCTC C | 21 |
| (5) | AACAAGATGA AATCTAGAAA ATCTTG | 26 |
| (6) | CAAGATTTTC TAGATTTCAT CTTGTT | 26 |
| (7) | TTCGTATTGC ACAGCCGAAG T | 21 |
| (8) | CAGCTTTGTG GGAATGTGGA TG | 22 |
| (9) | GCATTGAATA TTTGGGATCG TTT | 23 |
| (10) | GTTGAGTTGT ATACAGATAC TACTTTTGC | 29 |
| (11) | TTGGAGTGCT GGAGCTCGTG | 20 |
| (12) | GCTTTGTGGG AATGTGGA | 18 |
| (13) | CAGCTTTGTG GGAATGTGGA TGCGCG | 26 |
| (14) | TTGGAGTGCT GGAGCTC | 17 |
| (15) | TGCTTGGAGT GCTGGAGCTC GTG | 23 |

```
(16)  TTTAGGCGCT  TCTTTCCAAT  AC                                      22

(17)  GCATTGAATA  TTTGGGATC                                           19

(18)  GCATTGAATA  TTTGGGATCG  TTTTGATG                                28

(19)  GTTGAGTTGT  ATACAGATAC  TACT                                    24

(20)  GTTGAGTTGT  ATACAGATAC  TACTTTTGCT  TGG                         33

21)   CTTCCTTCTC  TCGTTTCCTT  GCAATTGAAC  AAGATGAAAT  CTAGAAAATC     50

      TTGCGGTATT  GCAGTAGGAA  CAACTATTGT                             80
```

Probes of the invention are useful for amplifying nucleic acid sequences which hybridize to the probes. These nucleic acid sequences could then be cloned and used to express MOMP or an amino acid sequence chemically synthesized. This amino acid sequence could then be used to make antibodies. Due to the degeneracy of the genetic code (many amino acids are selected by more than one codon) variations in the probe sequence will result in the same antibody specificity. Such antibodies can be polyclonal or monoclonal.

Probes of the invention can be deoxyribonucleic acid (DNA) or ribonucleic acid (RNA). DNA is composed of nucleotides based on purines (adenine and guanine) and pyrimidines (cytosine and thymine), with adenine base pairing (complementary) to thymine and quanine base pairing to cytosine. RNA is similar to DNA, except for the sugar differences (ribose instead of deoxyribose) and the base uracil, which is present instead of thymine. Also, modified nucleosides can be used in constructing probes of the invention. Modified nucleosides are typically used to obtain a probe capable of withstanding more stringent hybridization conditions, capable of easier detection, and the like. Preferably the probes are DNA.

The present invention can detect the presence of Chlamydia trachomatis in a variety of samples. Samples can include clinical specimens such as fecal material, blood, sputum, saliva, urine, plaque samples, tissue samples, fixed tissues, and tissue culture monolayers obtained by standard techniques such as lavage, scraping, or biopsy. The location of the C. trachomatis is not key to the invention, the ability to specifically detect C. trachomatis is key to the invention.

Samples obtained for use with probes of the invention can be utilized directly or amplified before using probes of the invention. A variety of amplification methods are available. For example, polymerase chain reaction (PCR), PCR Technology, H.A. Erlich, Ed. (Stockton Press, New York, NY, 1989), transcription-based amplification system (TAS), Proc. Natl. Acad. Sci. USA.**86**:1173 (1989), ligation amplification reaction (LAR), Genomics **4**:560 (1989), ligase based amplification system (LAS), Gene **89**:117 (1990), and Q B replicase, Infect. Dis. **162**:13 (1990). The goal of any sample preparation is to eliminate false positives and improve sensitivity. Such a goal is obtained by taking into account the way samples are prepared, the specific activity of labelled probes, and the selection of a medium or substance in which the sample is prepared.

Probes of the invention can be prepared in a variety of ways and therefore are not limited to any particular preparation means. Suitable means for preparing the probes of the invention include using replication vectors and cloning the probe as a library, followed by appropriate screening procedures, and growing the vector in a suitable host. Purification and isolation will result in the probe being separated from the vector with select restriction enzymes. Preferably the probes are synthesized using commercially available methods and equipment. For example, the solid phase phosphoramidite method can be used to produce the probes of the invention. C. Caruthers, et al., Cold Spring Harbour Symp. Quant. biol., **47**:411-418 (1982), and Adams, et al., J. Am. Chem. Soc., 105:601 (1983).

Typically, for synthesis of nucleic acid probes a nucleoside is converted to a phosphoramidite derivative that is appropriately protected for synthesis. See S. P. Adams et al., J. Am. Chem. Soc., **105**:661 (1983), L. J. McBride and M. H. Caruthers, Tetrahedron Lett., **24**:245 (1983), and N. D. Sinha et al., Nucleic Acids Res., **12**:4539 (1984). Methods for incorporating phosphoramidites into a nucleic acid strand include the use of solid phase, solution phase, triesters, and H-phosphonate intermediates as generally illustrated by Froehler et al.,

Nuc. Acids Res., **14**:5399 (1986), Narang et al, Methods Enz., **68**:90 (1979) and Ogilvie, K.K. et al., Proc., Natl. Acad. Sci. U.S.A., **85**:5764 (1988)

Probes of the invention can be conventionally synthesized by the modified phosphotriester method using fully protected deoxyribonucleotide building blocks. Such synthetic methods can be carried out in substantial accordance with the procedure of Itakura et al., 1977, Science, **198**:1056 and Crea et al., (1978), Proc. Nat. Acad. Sci. U.S.A., **75**:575, Hsiung et al., (1983), Nucleic Acid Research, **11**:3227, and Narang et al., (1980), Methods in Enzymology, **68**:90. In addition to manual procedures, the probes can be synthesized using automated synthesizers, such as the Systec 1450A or ABI 380A Synthesizers.

Probes of the invention can be utilized with naturally occurring sugar-phosphate backbones as well as modified backbones including phosphorothioates, dithionates, alkyl phosphonates and $\alpha$-nucleotides. Modified sugar-phosphate backbones are generally illustrated by Miller and T'so, Ann. Reports Med. Chem., **23**:295 (1988) and Moran et al., Nuc. Acids Res., **14**:5019 (1987).

Use of probes in detection methods include Northern blots (RNA detection), Southern blots (DNA detection), western blots (protein detection), dot blots (DNA, RNA, or protein detection), and Slot blots (DNA, RNA or protein). Other detection methods include kits containing probes on a dipstick setup and the like.

To detect hybrid molecules formed from using the probes of the invention, typically a detectable marker is added to one of the probes. Probes can be labelled by several methods. Probes can be radiolabelled and detected by audioradiography. Such labels for audioradiography include $^3$H, $^{125}$I, $^{35}$S, $^{14}$C, and $^{32}$P. Typically the choice of radioactive isotopes depends on research preferences involving ease of synthesis, stability, and half lives of the isotopes. Other detectable markers include ligands, fluorophores, chemiluminescent agents, electrochemical via sensors, time-resolved fluorescence, enzymes, and antibodies. For example, an antibody can be labelled with a ligand. Other detectable markers for use with probes of the invention include biotin, radionucleotides, enzyme inhibitors, co-enzymes, luciferins, paramagnetic metals, spin labels, and monoclonal antibodies. The choice of label dictates the manner in which the label is bound to the probe.

Radioactive nucleotides can be incorporated into probes of the invention by several means. Such means include nick translation of double-stranded probes, copying single-stranded M13 plasmids having specific inserts with the Klenow fragment of DNA polymerase I of E. coli or other such DNA polymerase in the presence of radioactive dNTP, transcribing cDNA from RNA templates using reverse transcriptase in the presence of radioactive dNTP, transcribing RNA from vectors containing strong promoters such as SP6 promoters or T7 promoters using SP6 or T7 RNA polymerase in the presence of radioactive rNTP, tailing the 3' ends of probes with radioactive nucleotides using terminal transferase, and by phosphorylation of the 5' ends of probes using gamma$^{32}$P ATP and polynucleotide kinase.

Non-radioactive probes of the invention can be labelled by indirect means. For example, a ligand molecule can be covalently bound to a probe of the invention. The ligand can then bind to an anti-ligand molecule which is either inherently detectable or covalently bound to a signal system such as a detectable enzyme, a flourescent compound, or chemiluminescent compound.

Probes of the invention can also be conjugated directly to signal generating compounds by such means as conjugation with an enzyme. Suitable enzymes for labels include hydrolases, particularly phosphatases, esterases, glycosidases, and oxidoreductases such as peroxidases. Fluorescent compounds include fluorescein and derivatives thereof, rhodamine and derivatives thereof, dansyl, umbelliferone, and the like.

Various hybridization conditions can be employed when using probes of the invention. The particular hybridization technique is not essential to the invention. Hybridization techniques are generally described in "Nucleic Acid Hybridization, A Practical Approach", edited by Hanes, B.D. and Higgins, S.J., IRL Press, 1985, Gall and Pardue (1969), Proc. Natl. Acad. Sci., USA,**63**:378-383, John Burnsteil and Jones (1969) Nature **223**:582-587, and Southern, E., J. Mol. Biol. **98**:503 (1975). With routine experimentation the conditions which permit satisfactory hybridization are easily obtained.

The correct hybridization complex can be detected in accordance with the label used with the probes. Therefore, the choice of label will guide the choice of detection methods. For example, when the label is a hapten or antigen, the hybridization complex can be detected using antibodies. Typically, the antibody will be attached to a flourescent or enzyme molecule which can be detected. Tijssen, P., "Practice in Theory of Enzyme Immunoasase, Laboratory Techniques in Biochemistry and Molecular Biology", Burdon, R. H., Van Knippenberg, P.H., Eds., Elsevier, 1985, PP.9-20. When the label is radioactive, the hybridization complex is exposed to X-ray film. Where the label is fluorescent, the sample is detected by irradiation with light of a particular wavelength. Enzyme labels are detected by incubation with appropriate substrate.

In addition to using probes of the invention for detecting sequences of C. trachomatis, the probes of the invention can be used for isolating sequences of C. trachomatis. Sometimes, like in many diagnostic applications, merely detecting the presence of a sequence of C. trachomatis in a sample is all that is desired. However, there are situations where it is desirable to isolate a sequence of C. trachomatis. For example, a sequence can be isolated as a preliminary step to separate extraneous DNA and then the specific isolated DNA sequence is amplified using DNA amplification techniques.

Probes of the invention are also useful for amplification of C. trachomatis sequences. For example, probes of the invention can be used as primers in a variety of amplification protocols such as polymerase chain reaction, transcription-based amplification system, ligation amplification reaction, ligase based amplification system and QB replicase (referenced above).

The probes of the invention can be conveniently provided in the form of a kit. The kits can also comprise probes in which detection can also be specific, for example, the probes can be designed for fluorescence, radioactive, and chemiluminescence detection.

The following examples illustrate the specific embodiments of the invention described in this document. As would be apparent to skilled artisans, various changes and modifications are possible and are contemplated within the scope of the invention described.

## EXAMPLES

## EXAMPLE I

### A. Materials

1. Salmon sperm carrier DNA, 1 mg/ml in sterile water.
2. 20X Standard Saline Citrate (SSC), 1X = 0.15 M NaCl, 0.015 M sodium citrate pH 7.
3. Wash buffer = 4X SSC, 5X Denhardt's solution, 0.1% SDS.
4. 10% Sodium dodecyl sulphate (SDS).
5. 50X Denhardt's solution, IX = 0.02% bovine serum albumin (BSA), 0.02% polyvinylpyrrolidone (PVP), and 0.02% ficoll-400 in sterile water and 0.45 $\mu$ filtered.
6. Major outer membrane protein (MOMP) target DNA.
7. Detector probe CT-27-AP (Seq ID No: 3 conjugated to alkaline phosphatase)
8. Capture probe CT-48-LCBiotin (Seq ID No: 6 conjugated to LC-biotin)
9. 3X Hybridization Buffer Mix = 15X Denhardt's, 12X SSC.
10. Assay Buffer = 1.0 M Diethanolamine, pH 9.8, 1 mM $MgCl_2$.
11. Substrate Mix = 5 mM p-nitrophenyl phosphate in Assay Buffer.
12. Biotin-Magnetic Particles (Advanced Magnetics Inc.) were washed 2X with 50 mM sodium phosphate pH 7.5, 5X Denhardt's then coated with streptavidin as follows. The particles were incubated in 1 mg/ml streptavidin dissolved in 50 mM sodium phosphate, pH 7.5 for 30 minutes. The particles were then washed 2X with Wash Buffer and resuspended in 50 mM sodium phosphate, 5X Denhardt's solution.

### B. Prodcedure

1. MOMP target DNA in water is mixed with 5 ug salmon sperm carrier DNA and denatured by heating for 3 minutes at 95°C.
2. Hybridization buffer components, capture and detector probes are added to give a 75 ul volume containing 4X SSC, 5X Denhardt's, 0.5% SDS, 10 pmol CT-48-LCBiotin, 10 pmol CT-27-AP.
3. The hybridization reaction is incubated 5 minutes at 50°C and then allowed to cool to room temperature for several minutes.
4. 100 ul of streptavidin-coated biotin magnetic particles are added, mixed well and incubated for 5 minutes at room temperature to allow capture of the hybridized probe-target complex.
5. Magnetic separation is used to separate the magnetic particles and the supernatant is aspirated. The particles are washed 3X with 400 ul of Wash Buffer.
6. The washed particles are suspended in 200 ul of substrate mix and incubated for 1 hour at 37°C.
7. The particles are separated and the absorbance of the supernatant at 405 nm is measured spectrophotometrically.

### C. Results

| Femto mole MOMP Target | 40 | 20 | 5 | 2.5 | 0.625 | 0 |
|---|---|---|---|---|---|---|
| Absorbance 405 nm | 1.38 | 0.65 | 0.20 | 0.13 | 0.078 | 0.042 |

The target DNA sequence hybridizes with the capture (CT-48-LC Biotin) and detector (CT-27 AP) probes during the five minute incubation at 50°C. This complex is then bound to the streptavidin-coated biotin magnetic particles and is separated from the excess probe reagents. Increasing the target DNA in the assay causes an increase in the amount of detector probe bound. In the detection part of the assay the alkaline phosphatase label attached to the detector probe converts a substrate into a yellow product absorbing at 405nm. The absorbance generated corresponds to the amount of detector probe and therefore, also depends on the amount of target DNA in the sample. The results demonstrate a linear increase in absorbance at 405 nm with increasing amounts of target DNA.

EXAMPLE II

Polymerase Chain Reaction (PCR) amplification of MOMP DNA from Chlamydia trachomatis.

A. Materials

1. Chlamydia trachomatis elementary bodies; 15 serovars obtained from Washington Research Foundation.
2. MOMP oligonucleotides; oligo Sequence ID No: 1 and Sequence ID No: 2
3. PCR Perkin-Elmer Thermal Cycler
4. PCR Reaction Buffer: 50mM KCl, 10mM Tris, pH8.3, 1.5mM $MgCl_2$, 0.1% gelatin, 1μM oligo Sequence ID No: 1, 1μM oligo Sequence ID No: 2, 0.25mM deoxyATP, 0.25mM deoxyGTP, 0.25mM deoxyGTP, 0.25mM deoxyTTP, 5 units Taq polymerase.

B. Procedure for PCR Amplification of MOMP DNA:

1. Chlamydia trachomatis elementary bodies, serovar L2 were serially diluted into PCR reaction buffer.
2. Samples were temperature cycled as follows: heated at 94°C for 1 minute, cooled to 37°C for 2 minutes and heated to 72°C for 3 minutes. This sequence of temperature cycling was repeated 25 times.
3. Following PCR reaction, the samples were analyzed by agarose gel electrophoresis using ethidium bromide staining. Results demonstrate the presence of Chlamydia MOMP target.
4. PCR Amplified MOMP DNA was detectable using the magnetic bead assay described above.

EXAMPLE III

PCR Amplification of MOMP DNA from all 15 serovars of Chlamydia trachomatis using oligonucleotide Sequence ID No: 1 and Sequence ID No: 2 and oligonucleotide Sequence ID No: 1 and Sequence ID No: 4
1. Chlamydia trachomatis elementary bodies from the following serovars were diluted in substantial accordance with the teachings of Example II: L1,L2,L3,A,B,Ba,C,D,E,F,G,H,I,J,K
2. PCR reactions were performed as described in example II, above, using oligos Sequence ID No: 1 and Sequence ID No: 2 and the amplified MOMP gene was detected by agarose gel electrophoresis.
3. The same MOMP gene was detected from all 15 serovars of Chlamydia trachomatis using oligos Sequence ID No: 1 and Sequence ID No: 4 and the following reaction conditions: 50mM KCl, 10mM Tris, pH8.3, 3mM $MgCl_2$, 0.3μM oligo Sequence ID No: 1, 0.3μM oligo Sequence ID No: 4, 0.5mM each of the four deoxynucleotides (dATP, dGTP, dCTP, dTTP), 100 mM 2-mercaptoethanol and 5 units Tag polymerase. Reactions contained 1 x $10^6$ elementary bodies and were temperature cycled 25 times at 1 minute 94°C, 1 minute at 55°C, and 1 minute at 72°C.
4. Specific amplification of the MOMP gene from each of the 15 serovars was detected by agarose gel electrophoresis and ethidium bromide staining.

EXAMPLE IV

PCR Amplification of the MOMP gene from Clinical Samples.
The oligo set Sequence ID No: 1 and Sequence ID No: 4 was used to evaluate 66 clinical samples (cervical swabs) previously determined to be Chlamydia trachomatis infected using conventional Chlamydia cell culture assays.
1. Clinical samples were treated with 0.17μg/μL proteinase K, 0.8% NP40 at 65°C for 1 hour. Proteinase K was inactivated by heating for 10 minutes at 95°C.
2. Samples were subjected to PCR amplification using the reaction conditions described in Example III with the exceptions that 4.5mM $MgCl_2$ was used and the samples temperature cycled for 40 cycles.
3. Samples were analyzed for MOMP gene amplification using agarose gel electrophoresis and ethidium bromide staining.

Results:     66 positive samples assayed

49 samples were positive (74%)

10 samples were indeterminant (15%)

7 samples were negative (10.6%)

A sample was determined to be positive if the diagnostic amplified band was clearly visible after electrophoresis and ethidium bromide staining. The negative samples failed to display the diagnostic band under the same conditions. Those samples deemed "indeterminate" were found to contain potent inhibitors of PCR and were unamplifiable.

Although the invention has been described with respect to specific modifications, the details thereof are not to be construed as limitations, for it will be apparent that various equivalents, changes and modifications may be resorted to without departing from the spirit and scope thereof, and it is understood that such equivalent embodiments are to be included therein.

SEQUENCE LISTING

(1) GENERAL INFORMATION:

    (i) APPLICANT: Malinowski, Douglas P.
                  Jurgensen, Stewart R.
                  Frasier, Melinda S.

   (ii) TITLE OF INVENTION: PROBES TO CHLAMYDIA TRACHOMATIS

  (iii) NUMBER OF SEQUENCES: 21

   (iv) CORRESPONDENCE ADDRESS:
        (A) ADDRESSEE: Richard J. Rodrick
        (B) STREET: 1 Becton Drive
        (C) CITY: Franklin Lakes
        (D) STATE: New Jersey
        (E) COUNTRY: USA
        (F) ZIP: 07417-1880

    (v) COMPUTER READABLE FORM:
        (A) MEDIUM TYPE: Floppy disk
        (B) COMPUTER: IBM PC compatible
        (C) OPERATING SYSTEM: PC-DOS/MS-DOS
        (D) SOFTWARE: PatentIn Release #1.0, Version #1.25

   (vi) CURRENT APPLICATION DATA:
        (A) APPLICATION NUMBER:
        (B) FILING DATE:
        (C) CLASSIFICATION:

 (viii) ATTORNEY/AGENT INFORMATION:
        (A) NAME: Stierwalt, Brian K.
        (B) REGISTRATION NUMBER: 33,213
        (C) REFERENCE/DOCKET NUMBER: P-1487

   (ix) TELECOMMUNICATION INFORMATION:
        (A) TELEPHONE: 201-847-5317
        (B) TELEFAX: 201-848-9228

(2) INFORMATION FOR SEQ ID NO:1:

    (i) SEQUENCE CHARACTERISTICS:
        (A) LENGTH: 22 base pairs
        (B) TYPE: nucleic acid
        (C) STRANDEDNESS: double

    (xi) SEQUENCE DESCRIPTION: SEQ ID NO:1:

ATGAAAAAAC TCTTGAAATC GG                                              22

(2) INFORMATION FOR SEQ ID NO:2:

        (i) SEQUENCE CHARACTERISTICS:
            (A) LENGTH: 25 base pairs
            (B) TYPE: nucleic acid
            (C) STRANDEDNESS: double


    (xi) SEQUENCE DESCRIPTION: SEQ ID NO:2:

GCATTTACGT GAGCTGCTCT CTCAT                                           25

(2) INFORMATION FOR SEQ ID NO:3:

        (i) SEQUENCE CHARACTERISTICS:
            (A) LENGTH: 22 base pairs
            (B) TYPE: nucleic acid
            (C) STRANDEDNESS: double


    (xi) SEQUENCE DESCRIPTION: SEQ ID NO:3:

GGAGATCCTT GCGATCCTTG CC                                              22

(2) INFORMATION FOR SEQ ID NO:4:

        (i) SEQUENCE CHARACTERISTICS:
            (A) LENGTH: 21 base pairs
            (B) TYPE: nucleic acid
            (C) STRANDEDNESS: double


    (xi) SEQUENCE DESCRIPTION: SEQ ID NO:4:

GCAAAGATCG CAAGGATCTC C                                               21

(2) INFORMATION FOR SEQ ID NO:5:

        (i) SEQUENCE CHARACTERISTICS:
            (A) LENGTH: 26 base pairs
            (B) TYPE: nucleic acid
            (C) STRANDEDNESS: double

        (xi) SEQUENCE DESCRIPTION: SEQ ID NO:5:

AACAAGATGA AATCTAGAAA ATCTTG                                          26

(2) INFORMATION FOR SEQ ID NO:6:

        (i) SEQUENCE CHARACTERISTICS:
            (A) LENGTH: 26 base pairs
            (B) TYPE: nucleic acid
            (C) STRANDEDNESS: double

        (xi) SEQUENCE DESCRIPTION: SEQ ID NO:6:

CAAGATTTTC TAGATTTCAT CTTGTT                                          26

(2) INFORMATION FOR SEQ ID NO:7:

        (i) SEQUENCE CHARACTERISTICS:
            (A) LENGTH: 21 base pairs
            (B) TYPE: nucleic acid
            (C) STRANDEDNESS: double

        (xi) SEQUENCE DESCRIPTION: SEQ ID NO:7:

TTCGTATTGC ACAGCCGAAG T                                               21

(2) INFORMATION FOR SEQ ID NO:8:

        (i) SEQUENCE CHARACTERISTICS:
            (A) LENGTH: 22 base pairs
            (B) TYPE: nucleic acid
            (C) STRANDEDNESS: double

(xi) SEQUENCE DESCRIPTION: SEQ ID NO:8:

CAGCTTTGTG GGAATGTGGA TG                22

(2) INFORMATION FOR SEQ ID NO:9:

    (i) SEQUENCE CHARACTERISTICS:
        (A) LENGTH: 23 base pairs
        (B) TYPE: nucleic acid
        (C) STRANDEDNESS: double

(xi) SEQUENCE DESCRIPTION: SEQ ID NO:9:

GCATTGAATA TTTGGGATCG TTT                23

(2) INFORMATION FOR SEQ ID NO:10:

    (i) SEQUENCE CHARACTERISTICS:
        (A) LENGTH: 29 base pairs
        (B) TYPE: nucleic acid
        (C) STRANDEDNESS: double

(xi) SEQUENCE DESCRIPTION: SEQ ID NO:10:

GTTGAGTTGT ATACAGATAC TACTTTTGC                29

(2) INFORMATION FOR SEQ ID NO:11:

    (i) SEQUENCE CHARACTERISTICS:
        (A) LENGTH: 20 base pairs
        (B) TYPE: nucleic acid
        (C) STRANDEDNESS: double

(xi) SEQUENCE DESCRIPTION: SEQ ID NO:11:

TTGGAGTGCT GGAGCTCGTG                20

(2) INFORMATION FOR SEQ ID NO:12:

    (i) SEQUENCE CHARACTERISTICS:
        (A) LENGTH: 18 base pairs
        (B) TYPE: nucleic acid
        (C) STRANDEDNESS: double


    (xi) SEQUENCE DESCRIPTION: SEQ ID NO:12:

GCTTTGTGGG AATGTGGA                              18

(2) INFORMATION FOR SEQ ID NO:13:

    (i) SEQUENCE CHARACTERISTICS:
        (A) LENGTH: 26 base pairs
        (B) TYPE: nucleic acid
        (C) STRANDEDNESS: double


    (xi) SEQUENCE DESCRIPTION: SEQ ID NO:13:

CAGCTTTGTG GGAATGTGGA TGCGCG                    26

(2) INFORMATION FOR SEQ ID NO:14:

    (i) SEQUENCE CHARACTERISTICS:
        (A) LENGTH: 17 base pairs
        (B) TYPE: nucleic acid
        (C) STRANDEDNESS: double


    (xi) SEQUENCE DESCRIPTION: SEQ ID NO:14:

TTGGAGTGCT GGAGCTC                              17

(2) INFORMATION FOR SEQ ID NO:15:

    (i) SEQUENCE CHARACTERISTICS:
        (A) LENGTH: 23 base pairs
        (B) TYPE: nucleic acid
        (C) STRANDEDNESS: double

EP 0 546 761 A1

(xi) SEQUENCE DESCRIPTION: SEQ ID NO:15:

TGCTTGGAGT GCTGGAGCTC GTG                                    23

(2) INFORMATION FOR SEQ ID NO:16:

    (i) SEQUENCE CHARACTERISTICS:
       (A) LENGTH: 22 base pairs
       (B) TYPE: nucleic acid
       (C) STRANDEDNESS: double

    (xi) SEQUENCE DESCRIPTION: SEQ ID NO:16:

TTTAGGCGCT TCTTTCCAAT AC                                     22

(2) INFORMATION FOR SEQ ID NO:17:

    (i) SEQUENCE CHARACTERISTICS:
       (A) LENGTH: 19 base pairs
       (B) TYPE: nucleic acid
       (C) STRANDEDNESS: double

    (xi) SEQUENCE DESCRIPTION: SEQ ID NO:17:

GCATTGAATA TTTGGGATC                                         19

(2) INFORMATION FOR SEQ ID NO:18:

    (i) SEQUENCE CHARACTERISTICS:
       (A) LENGTH: 28 base pairs
       (B) TYPE: nucleic acid
       (C) STRANDEDNESS: double

    (xi) SEQUENCE DESCRIPTION: SEQ ID NO:18:

GCATTGAATA TTTGGGATCG TTTTGATG                               28

16

(2) INFORMATION FOR SEQ ID NO:19:

    (i) SEQUENCE CHARACTERISTICS:
        (A) LENGTH: 24 base pairs
        (B) TYPE: nucleic acid
        (C) STRANDEDNESS: double

    (xi) SEQUENCE DESCRIPTION: SEQ ID NO:19:

GTTGAGTTGT ATACAGATAC TACT                    24

(2) INFORMATION FOR SEQ ID NO:20:

    (i) SEQUENCE CHARACTERISTICS:
        (A) LENGTH: 33 base pairs
        (B) TYPE: nucleic acid
        (C) STRANDEDNESS: double

    (xi) SEQUENCE DESCRIPTION: SEQ ID NO:20:

GTTGAGTTGT ATACAGATAC TACTTTTGCT TGG          33

(2) INFORMATION FOR SEQ ID NO:21:

    (i) SEQUENCE CHARACTERISTICS:
        (A) LENGTH: 80 base pairs
        (B) TYPE: nucleic acid
        (C) STRANDEDNESS: double

    (xi) SEQUENCE DESCRIPTION: SEQ ID NO:21:

CTTCCTTCTC TCGTTTCCTT GCAATTGAAC AAGATGAAAT CTAGAAAATC TTGCGGTATT  60

GCAGTAGGAA CAACTATTGT                      80

## Claims

1. A probe having a sequence consisting essentially of Seq ID No:1, Seq ID No: 2, Seq ID No: 3, Seq ID No: 4, Seq ID No: 5, Seq ID No: 6, Seq ID No: 7, Seq ID No: 8, Seq ID No: 9, Seq ID No: 10, Seq ID No: 11, Seq ID No: 12, Seq ID No: 13, Seq ID No: 14, Seq ID No: 15, Seq ID No: 16, Seq ID No: 17, Seq ID No: 18, Seq ID No: 19, Seq ID No: 20, or Seq ID No: 21, and the modified backbone, modified nucleotide, labelled forms, and ribonucleic acid forms thereof.

2. The probe of claim 1 which is the ribonucleic acid form thereof.

3. A method of amplifying a major outer membrane protein gene which comprises the use of one or more nucleic acid sequences selected from the sequences consisting essentially of Seq ID No:1, Seq ID No:

2, Seq ID No: 3, Seq ID No: 4, Seq ID No: 5, Seq ID No: 6, Seq ID No: 7, Seq ID No: 8, Seq ID No: 9, Seq ID No: 10, Seq ID No: 11, Seq ID No: 12, Seq ID No: 13, Seq ID No: 14, Seq ID No: 15, Seq ID No: 16, Seq ID No: 17, Seq ID No: 18, Seq ID No: 19, Seq ID No: 20, and Seq ID No: 21, and the modified backbone, modified nucleotide, labelled forms, and ribonucleic acid forms thereof.

4.   The method of claim 24 in which the nucleic acid sequence is the deoxyribonucleic acid form thereof.

5.   A method of detecting nucleic acid which comprises the use of one or more nucleic acid sequences selected from the sequences consisting essentially of Seq ID No:1, Seq ID No: 2, Seq ID No: 3, Seq ID No: 4, Seq ID No: 5, Seq ID No: 6, Seq ID No: 7, Seq ID No: 8, Seq ID No: 9, Seq ID No: 10, Seq ID No: 11, Seq ID No: 12, Seq ID No: 13, Seq ID No: 14, Seq ID No: 15, Seq ID No: 16, Seq ID No: 17, Seq ID No: 18, Seq ID No: 19, Seq ID No: 20, and Seq ID No: 21, and the modified backbone, modified nucleotide, labelled forms, and ribonucleic acid forms thereof.

6.   The method of claim 26 in which the nucleic acid sequence is the deoxyribonucleic acid form thereof.

7.   A method of amplifying nucleic acid and detecting the amplified product which comprises the use of one or more sequences selected from the sequences consisting essentially of Seq ID No:1, Seq ID No: 2, Seq ID No: 3, Seq ID No: 4, Seq ID No: 5, Seq ID No: 6, Seq ID No: 7, Seq ID No: 8, Seq ID No: 9, Seq ID No: 10, Seq ID No: 11, Seq ID No: 12, Seq ID No: 13, Seq ID No: 14, Seq ID No: 15, Seq ID No: 16, Seq ID No: 17, Seq ID No: 18, Seq ID No: 19, Seq ID No: 20, and Seq ID No: 21, and the modified backbone, modified nucleotide, labelled forms, and ribonucleic acid forms thereof.

8.   The method of claim 28 in which the nucleic acid sequence is the deoxyribonucleic acid form thereof.

9.   A kit comprising a nucleic acid sequence selected from the sequences consisting essentially of Seq ID No:1, Seq ID No: 2, Seq ID No: 3, Seq ID No: 4, Seq ID No: 5, Seq ID No: 6, Seq ID No: 7, Seq ID No: 8, Seq ID No: 9, Seq ID No: 10, Seq ID No: 11, Seq ID No: 12, Seq ID No: 13, Seq ID No: 14, Seq ID No: 15, Seq ID No: 16, Seq ID No: 17, Seq ID No: 18, Seq ID No: 19, Seq ID No: 20, and Seq ID No: 21, and the modified backbone, modified nucleotide, labelled forms, and ribonucleic acid forms thereof.

10.   The kit of claim 30 in which the nucleic acid sequence is the deoxyribonucleic acid form thereof.

11.   An antibody that recognizes the amino acid sequence encoded by the sequence selected from the sequence consisting essentially of Seq ID No:1, Seq ID No: 2, Seq ID No: 3, Seq ID No: 4, Seq ID No: 5, Seq ID No: 6, Seq ID No: 7, Seq ID No: 8, Seq ID No: 9, Seq ID No: 10, Seq ID No: 11, Seq ID No: 12, Seq ID No: 13, Seq ID No: 14, Seq ID No: 15, Seq ID No: 16, Seq ID No: 17, Seq ID No: 18, Seq ID No: 19, Seq ID No: 20, and Seq ID No: 21.

European Patent Office

# EUROPEAN SEARCH REPORT

Application Number

EP 92 31 0998

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 439 330 (IMPERIAL CHEMICAL INDUSTRIES) * page 16; claims * | 1-10 | C12Q1/68 |
| A | EP-A-0 293 079 (ALBERTA LMT) * the whole document * | 1-11 | |
| A | EP-A-0 420 260 (HOFFMAN LA ROCHE) * the whole document * | 1-10 | |
| A | WO-A-8 803 957 (GENPROBE INC.) * claims 123-156; example 11 * | 1-10 | |
| A | EP-A-0 336 412 (SCLAVO S.P.A.) | - | |
| A | WO-A-9 015 159 (GENE-TRAK SYSTEMS) | - | |

-----

|  |  |
|---|---|
|  | TECHNICAL FIELDS SEARCHED (Int. Cl.5) |
|  | C12Q |

The present search report has been drawn up for all claims

| Place of search | Date of completion of the search | Examiner |
|---|---|---|
| THE HAGUE | 31 MARCH 1993 | MOLINA GALAN E. |

EPO FORM 1503 03.82 (P0401)